# EUROPEAN PATENT SPECIFICATION

(11) **EP 3 370 551 B1**
(45) Date of publication and mention of the grant of the patent: **30.12.2020**
(21) Application number: 16781396.3
(22) Date of filing: 12.10.2016
(51) Int. Cl.: A24F 40/05, A24F 40/46

(54) **AN AEROSOL-GENERATING SYSTEM COMPRISING A VIBRATABLE ELEMENT**
AEROSOL-ERZEUGUNGSSYSTEM MIT EINEM SCHWINGUNGSFÄHIGEN ELEMENT
SYSTÈME DE GÉNÉRATION D'AÉROSOL COMPRENANT UN ÉLÉMENT CAPABLE DE VIBRER

(30) Priority: 02.11.2015 EP 15192636
(43) Date of publication of application: 12.09.2018
(60) Divisional application: 20199094.2
(73) Proprietor: Philip Morris Products S.A., 2000 Neuchâtel (CH)
(72) Inventor: ZUBER, Gerard, CH-1055 Froideville (CH); BONNELY, Samuel, CH-2036 Cormondrèche (CH)
(74) Representative: Spencer, James Michael
(86) International application number: PCT/EP2016/074425
(87) International publication number: WO 2017/076590

(56) References cited:
- EP-A1- 1 005 917
- EP-A1- 1 736 065
- EP-A1- 2 340 729
- WO-A1-2004/103478
- WO-A1-2015/117700
- US-A1- 2012 048 266

## Description

The present invention relates to aerosol-generating systems, cartridges for aerosol-generating systems and atomisers comprising a vibratable element for atomising a liquid aerosol-forming substrate. The aerosol-generating system may be an electrically operated smoking system.

One type of aerosol-generating system is an electrically operated smoking system. Electrically operated smoking systems typically use a liquid aerosol-forming substrate which is atomised to form an aerosol. Electrically operated smoking systems typically comprises a power supply, a liquid-storage portion for holding a supply of liquid aerosol-forming substrate and an atomiser. A common type of atomiser used in electronically operated smoking systems comprises a coil of heater wire wound around an elongate wick soaked in liquid aerosol-forming substrate.

Other known types of atomiser use ultrasonic vibrations, rather than heat, to atomise a liquid substrate. There are two main types of atomiser that use ultrasonic vibrations to atomise a liquid substrate, which are referred to herein as 'active' and 'passive' ultrasonic atomisers. 'Passive' ultrasonic atomisers use an oscillating element to transmit vibrations to a liquid substrate. The vibrations generate pressure waves in the liquid substrate that push the substrate through a fine mesh or a narrow region to atomise the liquid. 'Active' ultrasonic atomisers use a vibrating mesh through which a liquid substrate is drawn and atomised by the vibrations. Examples of systems comprising atomisers which use vibrations to atomise a liquid substrate are described in: European patent application publication number EP 1005917 A1, in the name of Microflow Engineering S.A.; International patent application publication number WO 2004/103478 A1, in the name of Collins et al.; and United States patent application publication number US 2012/0048266 A1, in the name of Alelov.

Ultrasonic atomisers may produce aerosols having a more consistent droplet size than atomisers that use heat to atomise a liquid substrate. Ultrasonic atomisers may also generate aerosols having a lower temperature than atomisers that use heat to atomise a liquid substrate. However, a problem with most known ultrasonic atomisers is that they are not able to atomise highly viscous liquids. In addition, most known ultrasonic atomisers for use in electrically operated smoking systems may not generate an aerosol at a temperature that provides a user with a mouthfeel that is similar to that of the smoke from a conventional cigarette or cigar.

It would be desirable to provide an aerosol-generating system that ameliorates these problems. It would be desirable to provide an aerosol-generating system that is capable of atomising a range of liquid aerosol-forming substrates, having a range of viscosities. It would also be desirable to provide an electrically operated smoking system having an improved atomiser.

In a first aspect of the present invention, there is provided an aerosol-generating system comprising a liquid-storage portion comprising a housing for holding a liquid aerosol-forming substrate and heating means arranged to heat liquid aerosol-forming substrate. The heating means comprises means for heating a small amount of liquid aerosol-forming substrate at a time, the means for heating a small amount of liquid aerosol-forming substrate at a time comprising: a liquid passageway in communication with the liquid aerosol-forming substrate; and at least one heater arranged to heat liquid aerosol-forming substrate within the liquid passageway The aerosol-generating system further comprises a vibratable element comprising a plurality of passages through which heated liquid aerosol-forming substrate may pass to form an aerosol, and an actuator arranged to vibrate the vibratable element to generate the aerosol.

In use, a user may operate the system by operating a switch or by drawing on a mouthpiece of the system. The heating means may be activated, heating at least a portion of the liquid aerosol-forming substrate. The actuator may be activated, exciting vibrations in the vibratable element. The vibrations in the vibratable element may deform the vibratable element and the passages of the plurality of passages. Heated liquid aerosol-forming substrate may be received by the vibratable element at an inlet side. The deformation of the passages may draw the received, heated liquid aerosol-forming substrate into the passages and may eject aerosol droplets of the liquid aerosol-forming substrate from an opposing outlet side of the element, atomising the liquid aerosol-forming substrate.

The viscosity of a liquid aerosol-forming substrate may have a significant effect on the flow-rate of the liquid through the aerosol-generating system. Reducing the viscosity of the liquid aerosol-forming substrate may increase the flow-rate and increases the rate of atomisation. As used herein, the term 'rate of atomisation' describes the rate of generation of aerosol from the system. In other words, the 'rate of atomisation' is the difference between the initial mass of aerosol-forming substrate held in the liquid storage portion and the remaining aerosol-forming substrate held in the liquid storage portion divided by the atomisation time.

The heating means may heat the liquid aerosol-forming substrate and reducing the viscosity of the liquid aerosol-forming substrate. By heating the liquid aerosol-forming substrate before atomisation, the heating means may increase the rate of atomisation. Heating the aerosol-forming substrate and reducing the viscosity of the aerosol-forming substrate before atomisation may also increase the reliability of the system.

The heating means may heat the liquid aerosol-forming substrate to a consistent, predetermined temperature before atomisation. This enables atomisation of the liquid aerosol-forming substrate at a consistent viscosity, and may enable generation of an aerosol by the system at a consistent rate of atomisation. This may improve the user experience.

The viscosity of the liquid aerosol-forming substrate may have a significant effect on the droplet size of the aerosol generated by the system. Therefore, heating the liquid aerosol-forming substrate to a consistent, predetermined temperature before atomisation may facilitate generation of an aerosol having a consistent distribution of droplet sizes.

Heating the liquid aerosol-substrate to a temperature above ambient temperature before atomisation may also reduce the sensitivity of the system to fluctuations in ambient temperature and provide a user with a consistent aerosol at each use.

As used herein, the term 'droplet size' is used to mean the aerodynamic droplet size, which is the size of a spherical unit density droplet that settles with the same velocity as the droplet in question. Several measures are used in the art to describe aerosol droplet size. These include mass median diameter (MMD) and mass median aerodynamic diameter (MMAD). As used herein, the term 'mass median diameter (MMD)' is used to mean the diameter of a droplet such that half the mass of the aerosol is contained in small diameter droplets and half in large diameter droplets. As used herein, the term 'mass median aerodynamic diameter (MMAD)' is used to mean the diameter of a sphere of unit density that has the same aerodynamic properties as a droplet of median mass from the aerosol.

There are several methods of measuring droplet size that are well-known in the art, in particular using laser based light scattering devices and inertial impaction devices. Laser diffraction devices do not typically detect aerodynamic droplet size. Inertial impaction devices typically detect aerodynamic droplet size and allow the amount of liquid contained in the droplets to be calculated. Example inertial impaction devices include the glass multistage liquid impinger, the Anderson impactor, the high performance multistage liquid impinge and the twin stage impingers.

The mass median aerodynamic diameter (MMAD) of the droplets generated by the aerosol-generating system of the present invention may be between about 1 µm and about 10 µm, or the MMAD may be between about 1 µm and about 5 µm. The MMAD of the droplets may be equal to or less than 3 µm. The desired droplet size of the droplets generated by the aerosol-generating system of the present invention may be any MMAD described above. The desired droplet size (MMAD) may be equal to or less than 3 µm.

The heating means may be any suitable heating means capable of heating a liquid aerosol-forming substrate. The heating means may be an electrically operated heating means. The heating means may be a resistive heating means.

The heating means may be arranged on or within the housing of the liquid-storage portion. This may improve heat transfer between the heating means and the liquid aerosol-forming substrate.

The heating means may be arranged at the vibratable element. In particular, the heating means may be in a heat conductive relationship with the vibratable element.

The heating means may be substantially flat to allow for straightforward manufacture. As used herein, the term 'substantially flat' means formed in a single plane and not wrapped around or otherwise confirmed to fit a curved or other non-planar shape. A flat heating means may be easily handled during manufacture and provide for a robust construction.

The heating means may be of the type described in EP-B1-2493342. For example, the heating means may comprise one or more electrically conductive tracks on an electrically insulating substrate. The electrically insulating substrate may comprise any suitable material, and may be a material that is able to tolerate high temperatures (in excess of 300°C) and rapid temperature changes. An example of a suitable material is a polyimide film, such as Kapton®.

The heating means comprises means for heating a small amount of liquid aerosol-forming substrate at a time. The means for heating a small amount of liquid aerosol-forming substrate at a time may include, for example, a liquid passageway in communication with the liquid aerosol-forming substrate. The liquid aerosol-forming substrate may be forced into the liquid passageway by capillary force. The at least one heater may be arranged such that during use, only the small amount of liquid aerosol-forming substrate within the liquid passageway, and not the liquid within the housing, is heated. The heating means may comprise a coil substantially surrounding at least a portion of a liquid passageway. The heating means may comprise inductive heating means. Inductive heating means are described in more detail below, in relation to the cartridge.

The heating means may comprise the vibratable element. This may reduce the number of component parts of the system and facilitate straightforward manufacture. This may ensure that the portion of liquid aerosol-forming substrate to be atomised (i.e. the portion received at the vibratable element) is at the desired temperature and viscosity at the time that it is atomised. This may also enable the heating means to operate at a lower temperature without reducing the temperature or the viscosity of the liquid aerosol-forming substrate being atomised. This is because the heating means may heat a portion of the liquid aerosol-forming substrate, rather than all of the liquid aerosol-forming substrate held in the housing. Lowering the operating temperature of the heating means may reduce the power requirements of the system.

The aerosol-generating system may further comprise a control system configured to operate the heating means to heat liquid aerosol-forming substrate to a predetermined temperature. The predetermined temperature may be above ambient temperature. The predetermined temperature may be above room temperature. This may reduce the viscosity of the aerosol-forming substrate compared to the viscosity of the unheated aerosol-forming substrate. This may increase the rate of atomisation and may facilitate generation of an aerosol having desirable droplet sizes. This may reduce the sensitivity of the system to fluctuations in ambient temperature.

The predetermined temperature may be below the vaporisation temperature of the liquid aerosol-forming substrate. The predetermined temperature may be between 20°C and 80°C, or between 30°C and 60°C or between 35°C and 45°C. The predetermined temperature may be between 20°C and 30°C, 30°C and 40°C, 40°C and 50°C, 50°C and 60°C, 60°C and 70°C or 70°C and 80°C.

As used herein, the term ambient temperature' is used to mean the air temperature of the surrounding environment in which the aerosol-generating system is being used. Ambient temperatures typically corresponds to a temperature between about 10°C and 35°C. As used herein, the term 'room temperature' is used to mean a standard ambient temperature and pressure, typically a temperature of about 25 °C and an absolute pressure of about 100 kPa (1 atm).

The control system configured to operate the heating means may be separate of other control systems of the aerosol-generating system. The control system may be integral with other control system of the aerosol-generating system. The control system may comprise electric circuitry connected to the heating means and to an electrical power source. The electric circuitry may be configured to monitor the electrical resistance of the heating means and to control the supply of power to the heating means dependent on the electrical resistance of the heating means.

The electric circuitry may comprise a microprocessor, which may be a programmable microprocessor. The electric circuitry may comprise further electronic components. The electric circuitry may be configured to regulate a supply of power to the heating means. Power may be supplied to the heating means continuously following activation of the system or may be supplied intermittently, such as on a puff-by-puff basis. The power may be supplied to the heating means in the form of pulses of electrical current.

The control system may comprise an ambient temperature sensor, to detect the ambient temperature. The control system may comprise a temperature sensor within the liquid storage portion, to detect the temperature of the liquid-aerosol-forming substrate held in the housing of the liquid storage portion. The one or more temperature sensors may be in communication with control electronics of the aerosol-generating system to enable the control electronics to maintain the temperature of the liquid aerosol-forming substrate at the predetermined temperature. The one or more temperature sensors may be a thermocouple. The heating means may be used to provide information relating to the temperature. Temperature dependent resistive properties of the heating means may be known and used to determine the temperature of the at least one heater in a manner known to the skilled person.

The vibratable element may be a thin sheet. As used herein, 'thin' denotes a body having a thickness that is substantially smaller than the other dimensions of the body, such as length, width or diameter. The vibratable element may have a thickness of between about 0.1 mm and about 4.0 mm. The vibratable element may have a longitudinal length or diameter of between about 3 mm and about 60 mm.

As used herein, the term 'diameter' denotes the maximum dimension in the transverse direction of parts or portions of parts of the aerosol-generating system.

The vibratable element may be any suitable shape. The vibratable element may be substantially circular or elliptical. The vibratable element may be substantially triangular or square or any regular or irregular shape. The vibratable element may be substantially flat. The vibratable element may be curved. The vibratable element may be dome shaped. The vibratable element may be a substantially square plate. The vibratable element may be a substantially circular or elliptical disc.

The vibratable element may comprise a single piece of material. The vibratable element may comprise more than one piece of material. The vibratable element may be laminated. The vibratable element may comprise a metal or a metal alloy. The metal or metal alloy may be nickel, iron, titanium, copper or aluminium. The vibratable element may comprise a polymeric material. The vibratable element may comprise a ceramic material. The vibratable element may comprise a combination of materials.

The vibratable element may comprise an inlet side and an opposing outlet side, and each passage of the plurality of passages may extend between the inlet side and the outlet side.

The vibratable element may be reusable. The vibratable element may be disposable.

The passages of the plurality of passages are open passages that extend through the thickness of the vibratable element. The passages have open ends at the opposing inlet and outlet sides of the vibratable element. The passages may be formed in the vibratable element by any suitable method. Suitable known methods of forming the passages include electrolysis and high-speed laser drilling.

The passages may have any suitable shape. The passages may have a substantially circular or elliptical cross-section. The passages may have a substantially triangular or square or an irregularly shaped cross-section.

The passages may have a consistent diameter along their length. The passages may be substantially cylindrical. The passages may have a tapered shape with a width that narrows towards the outlet surface of the vibratable element. Providing passages with a larger diameter at the inlet side (i.e. the side receiving the liquid aerosol-forming substrate) than at the outlet side may facilitate uptake of the liquid-aerosol-forming substrate by the passageways. This may increase the rate of atomisation of liquid aerosol-forming substrate.

The diameter of the tapered passages may decrease continuously along the length of passages between the inlet and outlet sides. The diameter of the tapered passages may vary in one or more discrete step changes between the inlet and outlet sides. The tapered passages may be substantially frusto-conical, forming truncated cones. The tapered passages may be substantially truncated pyramids. The angle of taper may be constant along the length of the tapered passages. As used herein, the term 'angle of taper' is used to mean the angular deviation of the passage walls from the normal to the first or second surface of the vibratable element.

The passages may have a diameter at the outlet side of the vibratable element of between about 1 micrometre (µm) and 150 micrometres (µm), or between about 1 µm and 50 µm, or between about 1.5 µm and 10 µm. This may facilitate generation of aerosols having desirable droplet sizes. The passages may have any suitable diameter at the outlet side of the vibratable element to generate droplets having a desired droplet size. The desired droplet size (MMAD) may be equal to or less than 3 µm.

The passages may give rise to capillary action, so that in use, liquid aerosol-forming substrate to be atomised is drawn into the passages, increasing the contact area between the vibratable element and the liquid aerosol-forming substrate. Where the heating means comprises the vibratable element, this may improve conductive heat transfer between the vibratable element and the liquid aerosol-forming substrate.

The plurality of passages may form an array. The array of passages may have any suitable shape. For example, the passages may be arranged in a substantially circular array, a substantially elliptical array, a substantially square array or a substantially rectangular array. The passages may be regularly spaced across the array. The passages may be irregularly spaced across the array.

The array of passages may extend across the entire vibratable element. The array of passages may extend over a portion of the vibratable element. The array of passages may extend over a central portion of the vibratable element. The array may cover an area of between about 10% and about 100% of the area of the vibratable element, or between about 20% and about 80%, or between about 30% and about 70%. The area of the array of passages may be less than or equal to 25 mm². The array of passages may, for example, be rectangular and have dimensions of about 5 mm and about 2 mm. The array of passages may be substantially circular, having a diameter of between about 3 mm and about 60 mm.

The plurality of passages may comprise between about 100 and 10000 passages, or between about 1000 and 7000 passages, or between about 3000 and 5000 passages.

The actuator may be arranged at any suitable location with respect to the vibratable element. The actuator may be arranged to transmit vibrations to the vibratable element at the inlet side or the outlet side of the vibratable element. The actuator may be arranged to transmit vibrations to the vibratable element at the inlet side. The actuator may be arranged to transmit vibrations to the vibratable element at the outlet side. The actuator may be in direct contact with the vibratable element. The actuator may be secured to the vibratable element. The actuator may be secured to the vibratable element by pressure. The actuator may be bonded to the vibratable element. A transfer member may be provided between the actuator and the vibratable element to transfer vibrations from the actuator to the vibratable element.

The actuator may be arranged to vibrate the vibratable element in any suitable direction. The actuator may be arranged to vibrate the vibratable element in a thickness direction. As used herein, 'thickness direction' means a direction substantially parallel to the thickness of the vibratable element. This may facilitate deformation in the vibratable element that encourages movement of liquid aerosol-forming substrate through the passages.

The actuator may comprise one or more actuating elements. The one or more actuating elements may be any suitable shape. The one or more actuating elements may be substantially circular or elliptical. The one or more actuating elements may be substantially triangular, square or any regular or irregular shape. The one or more actuating elements may be annular. The one or more actuating elements may substantially circumscribe the plurality of passages of the vibratable element. By circumscribing the plurality of passages, the one or more actuating elements may not cover an open end of the passages. The one or more actuating elements may be substantially flat. The one or more actuating elements may have a thickness of between about 0.1 mm and 5.0 mm. The one or more actuating elements may be a substantially annular disc. The outer diameter of the annular disc may be between about 3 mm and about 60 mm and the inner diameter may be between about 2 mm and about 59 mm.

The actuator may be any type of actuator for exciting vibrations in the vibratable element. The actuator may comprise a piezoelectric transducer. The piezoelectric transducer may provide actuator that is sufficiently small, lightweight and easy to control for use in a handheld aerosol-generating system.

The piezoelectric transducer may comprise a monocrystalline material. The piezoelectric transducer may comprise quartz. The piezoelectric transducer may comprise a ceramic. The ceramic may comprise barium titanate (BaTiO₃). The ceramic may comprise lead zirconate titanate (PZT). The ceramic may include doping materials such as Ni, Bi, La, Nd or Nb ions. The piezoelectric transducer may be polarised. The piezoelectric transducer may be unpolarised. The piezoelectric transducer may comprise both polarised and unpolarised piezoelectric materials.

The aerosol-generating system may further comprise a control system configured to operate the actuator to excite vibrations in the vibratable element at a predetermined frequency. The predetermined frequency may be between about 20 kHz and about 1500 kHz, or between about 50 kHz and about 1000 kHz, or between about 100 kHz and about 500 kHz. This may provide a desired aerosol-output rate and a desired droplet size for a good user experience.

The control system configured to operate the actuator may be separate of other control systems of the aerosol-generating system. The control system may be integral with other control system of the aerosol-generating system. The control system may comprise electric circuitry connected to the actuator and to an electrical power source.

The electric circuitry may comprise a microprocessor, which may be a programmable microprocessor. The electric circuitry may comprise further electronic components. The electric circuitry may be configured to regulate a supply of power to the actuator. Power may be supplied to the actuator continuously following activation of the system or may be supplied intermittently, such as on a puff-by-puff basis. The power may be supplied to the actuator in the form of pulses of electrical current.

The liquid storage portion of the aerosol-generating system may comprise a housing that is substantially cylindrical, wherein an opening is at one end of the cylinder. The housing of the liquid storage portion may have a substantially circular cross section. The housing may be a rigid housing. As used herein, the term 'rigid housing' is used to mean a housing that is self-supporting. The rigid housing of the liquid-storage portion may provide mechanical support to the heating means.

The liquid storage portion may further comprise a carrier material within the housing for holding the aerosol-forming substrate.

The liquid aerosol-forming substrate may be adsorbed or otherwise loaded onto a carrier or support. The carrier material may be made from any suitable absorbent plug or body, for example, a foamed metal or plastics material, polypropylene, terylene, nylon fibres or ceramic. The liquid aerosol-forming substrate may be retained in the carrier material prior to use of the aerosol-generating system. The liquid aerosol-forming substrate may be released into the carrier material during use. The liquid aerosol-forming substrate may be released into the carrier material immediately prior to use. For example, the liquid aerosol-forming substrate may be provided in a capsule. The shell of the capsule may melt upon heating by the heating means and releases the liquid aerosol-forming substrate into the carrier material. The capsule may optionally contain a solid in combination with the liquid.

In one example, the liquid aerosol-forming substrate is held in capillary material. A capillary material is a material that actively conveys liquid from one end of the material to another. The capillary material may be advantageously oriented in the housing to convey liquid aerosol-forming substrate to the first side of the vibratable element. The capillary material may have a fibrous structure. The capillary material may have a spongy structure. The capillary material may comprise a bundle of capillaries. The capillary material may comprise a plurality of fibres. The capillary material may comprise a plurality of threads. The capillary material may comprise fine bore tubes. The capillary material may comprise a combination of fibres, threads and fine-bore tubes. The fibres, threads and fine-bore tubes may be generally aligned to convey liquid to the vibratable element. The capillary material may comprise sponge-like material. The capillary material may comprise foam-like material. The structure of the capillary material may form a plurality of small bores or tubes, through which the liquid can be transported by capillary action.

The capillary material may comprise any suitable material or combination of materials. Examples of suitable materials are a sponge or foam material, ceramic- or graphite-based materials in the form of fibres or sintered powders, foamed metal or plastics materials, a fibrous material, for example made of spun or extruded fibres, such as cellulose acetate, polyester, or bonded polyolefin, polyethylene, terylene or polypropylene fibres, nylon fibres or ceramic. The capillary material may have any suitable capillarity and porosity so as to be used with different liquid physical properties. The liquid aerosol-forming substrate has physical properties, including but not limited to viscosity, surface tension, density, thermal conductivity, boiling point and atom pressure, which allow the liquid to be transported through the capillary material by capillary action. The capillary material may be configured to convey the aerosol-forming substrate to the first surface of the vibratable element. The capillary material may extend into passages of the vibratable element.

The carrier material may abut the vibratable element. The carrier material may abut the vibratable element at the inlet side. The capillary material may abut the vibratable element. The liquid aerosol-forming substrate may be transported by capillary action from the liquid storage portion to the vibratable element. By providing capillary material in abutment with the inlet side of the vibratable element, liquid aerosol-forming substrate from the liquid-storage portion may be delivered to the vibratable element regardless of the orientation of the aerosol-generating system.

The aerosol-generating system may comprise liquid aerosol-forming substrate in the housing of the liquid-storage portion. The liquid aerosol-forming substrate is a substrate capable of releasing volatile compounds that can form an aerosol. The volatile compounds may be released by moving the liquid aerosol-forming substrate through the passages of the vibratable element.

The liquid aerosol-forming substrate may comprise nicotine. The nicotine containing liquid aerosol-forming substrate may be a nicotine salt matrix. The liquid aerosol-forming substrate may comprise plant-based material. The liquid aerosol-forming substrate may comprise tobacco. The liquid aerosol-forming substrate may comprise a tobacco-containing material containing volatile tobacco flavour compounds, which are released from the aerosol-forming substrate upon heating. The liquid aerosol-forming substrate may comprise homogenised tobacco material. The liquid aerosol-forming substrate may comprise a non-tobacco-containing material. The liquid aerosol-forming substrate may comprise homogenised plant-based material.

The liquid aerosol-forming substrate may comprise at least one aerosol-former. An aerosol-former is any suitable known compound or mixture of compounds that, in use, facilitates formation of a dense and stable aerosol and that is substantially resistant to thermal degradation at the temperature of operation of the system. Suitable aerosol-formers are well known in the art and include, but are not limited to: polyhydric alcohols, such as triethylene glycol, 1,3-butanediol and glycerine; esters of polyhydric alcohols, such as glycerol mono-, di- or triacetate; and aliphatic esters of mono-, di- or polycarboxylic acids, such as dimethyl dodecanedioate and dimethyl tetradecanedioate. Aerosol formers may be polyhydric alcohols or mixtures thereof, such as triethylene glycol, 1,3-butanediol and glycerine. The liquid aerosol-forming substrate may comprise other additives and ingredients, such as flavourants.

The aerosol-forming substrate may comprise nicotine and at least one aerosol former. The aerosol former may be glycerine. The aerosol-former may be propylene glycol. The aerosol former may comprise both glycerine and propylene glycol. The aerosol-forming substrate may have a nicotine concentration of between about 2% and about 10%.

The aerosol-forming substrate may have a dynamic viscosity (µ) at a temperature of 20°C of between about 0.4 mPa.S (0.4 mPI, 0.4 cP) and about 1000 mPa.S (1000 mPI, 1000 cP), or between about 1 mPa.S and about 100 mPa.S, or about 1.5 mPa.S and about 10 mPa.S.

The aerosol-generating system may comprise a power supply. The power supply may be a battery. The battery may be a Lithium based battery, for example a Lithium-Cobalt, a Lithium-Iron-Phosphate, a Lithium Titanate or a Lithium-Polymer battery. The battery may be a Nickel-metal hydride battery or a Nickel cadmium battery. The power supply may be another form of charge storage device such as a capacitor. The power supply may require recharging and be configured for many cycles of charge and discharge. The power supply may have a capacity that allows for the storage of enough energy for one or more smoking experiences; for example, the power supply may have sufficient capacity to allow for the continuous generation of aerosol for a period of around six minutes, corresponding to the typical time taken to smoke a conventional cigarette, or for a period that is a multiple of six minutes. In another example, the power supply may have sufficient capacity to allow for a predetermined number of puffs or discrete activations of the heating means and actuator.

The aerosol-generating system may be portable. The aerosol-generating system may have a size comparable to a conventional cigar or cigarette. The aerosol-generating system may have a total length between about 30 mm and about 150 mm. The aerosol-generating system may have an external diameter between about 5 mm and about 30mm.

The aerosol-generating system may comprise a housing. The housing may be elongate. The housing may comprise any suitable material or combination of materials. Examples of suitable materials include metals, alloys, plastics or composite materials containing one or more of those materials, or thermoplastics that are suitable for food or pharmaceutical applications, for example polypropylene, polyetheretherketone (PEEK) and polyethylene. The material may be light and non-brittle.

The housing may comprise a cavity for receiving the power supply. The housing may comprise a mouthpiece. The mouthpiece may comprise at least one air inlet and at least one air outlet. The mouthpiece may comprise more than one air inlet. One or more of the air inlets may reduce the temperature of the aerosol before it is delivered to a user and may reduce the concentration of the aerosol before it is delivered to a user.

The aerosol-generating system may comprise an aerosol-generating device and a cartridge. The cartridge may comprise the liquid storage portion. The cartridge may comprise the liquid storage portion and at least a portion of the heating means. The cartridge may comprise the liquid storage portion and the heating means. The cartridge may comprise the liquid storage portion, the heating means, the vibratable element and the actuator.

A cartridge for an aerosol-generating system may comprise a liquid storage portion comprising a housing for holding a liquid aerosol-forming substrate; and heating means arranged to heat liquid aerosol-forming substrate to a predetermined temperature.

According to a second aspect of the present invention, there is provided a cartridge for an aerosol-generating system, the cartridge comprising a liquid storage portion comprising a housing for holding a liquid aerosol-forming substrate; heating means arranged to heat liquid aerosol-forming substrate to a predetermined temperature; a vibratable element comprising a plurality of passages through which heated liquid aerosol-forming substrate may pass to form an aerosol; and an actuator arranged to vibrate the vibratable element to generate the aerosol.

The liquid storage portion, the heating means, the vibratable element and the actuator may comprise any features or be arranged in any configuration as described above in relation to the liquid storage portion, the heating means, the vibratable element and the actuator of the aerosol-generating system of the first aspect of the present invention. For example, the heating means may comprise the vibratable element.

The heating means may be substantially as described above in relation to the first aspect of the present invention. The heating means may be inductive heating means, such that no electrical contacts are formed between the cartridge and the device. The device may comprise an inductor coil and a power supply configured to provide high frequency oscillating current to the inductor coil. The cartridge may comprise a susceptor element positioned to heat the aerosol-forming substrate. As used herein, a high frequency oscillating current means an oscillating current having a frequency of between 500 kHz and 10 MHz.

The predetermined temperature may be above ambient temperature. The predetermined temperature may be above room temperature. The predetermined temperature may be below the vaporisation temperature of the liquid aerosol-forming substrate. The predetermined temperature may be any suitable temperature for a vibratable element and actuator arrangement in accordance with the present invention to generate droplets having a desired droplet size. The desired droplet size (MMAD) may be equal to or less than 3 µm. The predetermined temperature may be between 20°C and 80°C, or between 30°C and 60°C or between 35°C and 45°C. The predetermined temperature may be between 20°C and 30°C, 30°C and 40°C, 40°C and 50°C, 50°C and 60°C, 60°C and 70°C or 70°C and 80°C.

The cartridge may be removably coupled to the aerosol-generating device. The cartridge may be removed from the aerosol-generating device when the aerosol-forming substrate has been consumed. The cartridge may be disposable. The cartridge may be reusable. The cartridge may be refillable with liquid aerosol-forming substrate. The cartridge may be replaceable in the aerosol-generating device. The aerosol-generating device may be reusable.

The cartridge may be manufactured at low cost, in a reliable and repeatable fashion. As used herein, the term removably coupled' is used to mean that the cartridge and device can be coupled and uncoupled from one another without significantly damaging either the device or cartridge.

The cartridge may have a simple design. The cartridge may have a housing within which an aerosol-forming substrate is held. The cartridge housing may be a rigid housing. As used herein 'rigid housing' means a housing that is self-supporting. The housing may comprise a material that is impermeable to liquid.

The cartridge may comprise a lid. The lid may be peelable before coupling the cartridge to the aerosol-generating device. The lid may be piercable.

The aerosol-generating device may comprise a cavity for receiving the cartridge. The aerosol-generating device may comprise a cavity for receiving the power supply.

The aerosol-generating device may comprise the heating means. The aerosol-generating device may comprise at least a portion of the heating means. The aerosol-generating device may comprise the vibratable element. The aerosol-generating device may comprise the actuator. The aerosol-generating device may comprise one or more control systems of the aerosol-generating system. The aerosol-generating device may comprise the power supply. The power supply may be removably coupled to the aerosol-generating device.

The aerosol-generating device may comprise a mouthpiece. The mouthpiece may comprise at least one air inlet and at least one air outlet. The mouthpiece may comprise more than one air inlet.

The aerosol-generating device may comprise a piercing element for piercing the lid of the cartridge. The mouthpiece may comprise the piercing element. The mouthpiece may comprise at least one first conduit extending between the at least one air inlet and a distal end of the piercing element. The mouthpiece may comprise at least one second conduit extending between a distal end of the piercing element and the at least one air outlet. The mouthpiece may be arranged such that in use, when a user draws on the mouthpiece, air flows along an airflow pathway extending from the at least one air inlet, through the at least one first conduit, through a portion of the cartridge, through the at least one second conduit and exits the at least one outlet. This may improve airflow through the aerosol-generating device and enable the aerosol to be delivered to the user more easily.

The aerosol-generating system may comprise a temperature sensor. The temperature sensor may be adjacent to the cavity for receiving the cartridge. The temperature sensor may be in communication with the control electronics to enable the control electronics to maintain the temperature of the heating means at the predetermined operating temperature. The temperature sensor may be a thermocouple, or alternatively the at least one heater may be used to provide information relating to the temperature. The temperature dependent resistive properties of the at least one heater may be known and used to determine the temperature of the at least one heater in a manner known to the skilled person.

The aerosol-generating system may comprise a puff detector in communication with the control electronics. The puff detector may be configured to detect when a user draws on the mouthpiece. The control electronics may be configured to control power to the at least one heating element in dependence on the input from the puff detector.

The aerosol-generating system may comprise a user input, such as a switch or button. This enables the user to turn the system on. The switch or button may activate the heating means. The switch or button may initiate the aerosol generation. The switch or button may prepare the control electronics to await input from the puff detector.

In use, a user may insert a cartridge as described herein into the cavity of an aerosol-generating device as described herein. The user may attach the mouthpiece to the main body of the aerosol-generating device, which may pierce the cartridge with the piercing portion. The user may activate the device by pressing the button. The user may then draw on the mouthpiece, which draws air into the device through the one or more air inlets, the air then passes through over the vibratable element, entraining the atomised aerosol-forming substrate into the airflow, and exits the device through the air outlet in the mouthpiece to be inhaled by the user.

The aerosol-generating system may be an electrically operated smoking system. The aerosol-generating system may be an electronic cigarette.

The electrically operated smoking system may comprise liquid aerosol-forming substrates that, at ambient temperatures, are too viscous to be atomised by other ultrasonic atomisers. The heating means may reduce the viscosity of the liquid aerosol-forming substrate before atomisation.

According to a third aspect of the present invention, there is provided an atomiser for atomising a liquid aerosol-generating substrate to generate an aerosol, the atomiser comprising: heating means arranged to heat liquid aerosol-forming substrate; a vibratable element comprising a plurality of passages through which heated liquid aerosol-forming substrate may pass to form an aerosol; and an actuator arranged to vibrate the vibratable element to generate the aerosol. The vibratable element and the heating means may comprise any features or be arranged in any configuration as described above in relation to the vibratable element and the heating means of the aerosol-generating system of the first aspect of the present invention. For example, the heating means may comprise the vibratable element.

A kit of parts may be provided, comprising an aerosol-generating device, a cartridge and an atomiser, substantially as described above. An aerosol-generating system in accordance with the first aspect of the present invention may be provided by assembling the aerosol-generating device, the cartridge and the atomiser of the kit of parts. The components of the kit of parts may be removably connected. The components of the kit of parts may be interchangeable. Components of the kit of parts may be disposable. Components of the kit of parts may be reusable.

According to a fourth aspect of the present invention, there is provided a method of generating an aerosol, the method comprising: heating a liquid aerosol-forming substrate to a predetermined temperature; receiving liquid aerosol-forming substrate heated to the predetermined temperature at a vibratable element having a plurality of passages; and vibrating the vibratable element to pass liquid aerosol-forming substrate through the passages to form an aerosol. The method may be performed using an aerosol-generating system, a cartridge or an atomiser in accordance with other aspects of the present invention.

The method has all of the advantages described in relation to the other aspects of the present invention. Features of the vibratable element and variables, such as the predetermined temperature and frequency of oscillation of the vibratable element, may be the same as those described in relation to the other aspects of the present invention.

Features described in relation to one aspect of the invention may also be applicable to another aspect of the invention.

The invention will be further described, by way of example only, with reference to the accompanying drawings, in which:
Figure 1 shows a schematic illustration of a first embodiment of an aerosol-generating system according to the present invention;
Figure 2 shows a first embodiment of an atomiser for an aerosol-generating system according to the present invention; and
Figure 3 shows a second embodiment of an atomiser for an aerosol-generating system.

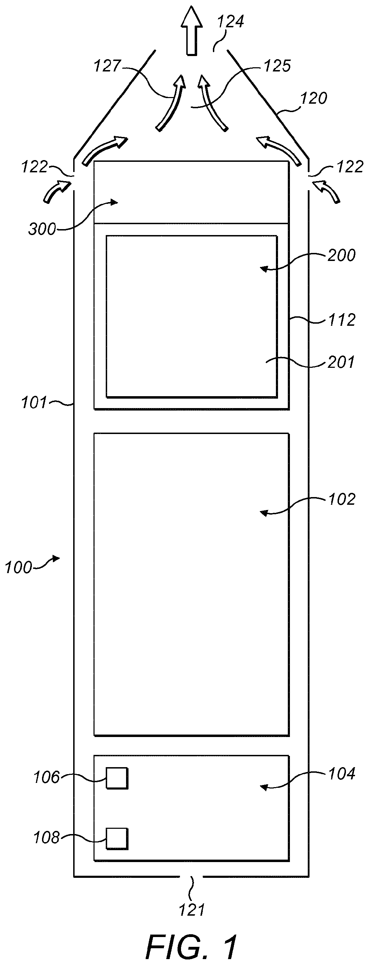

Figure 1 is a schematic view of a first embodiment of an aerosol-generating system according to the invention. Figure 1 is schematic in nature. In particular, the components shown are not necessarily to scale either individually or relative to one another. The aerosol-generating system comprises an aerosol generating device 100, which is preferably reusable, in cooperation with a cartridge 200, which is preferably disposable. In Figure 1, the system is an electrically operated smoking system.

The device 100 comprises a main body having a housing 101. The housing 101 is substantially circularly cylindrical and has a longitudinal length of about 100 mm and an external diameter of about 20 mm, comparable to a conventional cigar. In the device, there is provided an electric power supply in the form of battery 102 and control electronics 104. The main body housing 101 also defines a cavity 112 into which the cartridge 200 is received.

The cartridge 200 (shown in schematic form in Figure 1) comprises a rigid housing defining a liquid storage portion 201. The liquid storage portion 201 holds a liquid aerosol-forming substrate (not shown). The housing of the cartridge 200 is fluid impermeable but has an open end (not shown) that is coverable by a removable lid (not shown) when the cartridge is removed from the device 100. The lid may be removed from the cartridge 200 before insertion of the cartridge into the device. The cartridge 200 includes keying features (not shown) to ensure the cartridge 200 cannot be inserted into the device upside-down.

The device 100 also includes a mouthpiece portion 120. The mouthpiece portion 120 is connected to the main body housing 101 by a hinged connection in this example but any kind of connection may be used, such as a snap fitting or a screw fitting. The mouthpiece portion 120 comprises a plurality of air inlets 122, an air outlet 124 and an aerosol forming chamber 125, and an atomiser 300 mounted therein (shown schematically in Figure 1). Air inlets 122 are defined between the mouthpiece portion 120 and the main body housing 101 of the device 100 when the mouthpiece portion is in a closed position, as shown in Figure 1. An air-flow route 127 is formed from the air inlets 122 to the air outlet 124 via the aerosol forming chamber 125 and the atomiser 300, as shown in Figure 1 by the arrows.

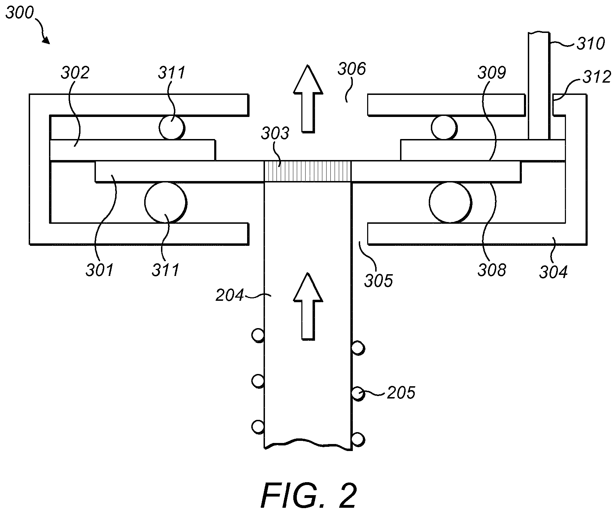

As shown in Figure 2, the atomiser 300 comprises a vibratable element 301 and actuator 302 housed inside an atomiser housing 304. Atomiser housing 304 comprises a hollow cylindrical box, having an inlet opening 305 and an outlet opening 306 arranged in co-axial alignment at opposite sides of the housing 304. The housing 304 is removably connected to the mouthpiece 120 of the device 100 by a screw thread connection (not shown). A male screw thread (not shown) is provided at an outer surface of the atomiser housing 304, that is complimentary to a female screw thread (not shown) on an inner surface of the mouthpiece 120. Atomiser 300 is removable from the mouthpiece portion 120 of the device for disposal or for cleaning.

Vibratable element 301 comprises a substantially circular aluminium disc, having a thickness of about 2 mm and a diameter of about 15 mm.

A plurality of passages 303 extends from an inlet side 308 to an opposing outlet side 309 of the vibratable element. The plurality of passages form an array having a substantially circular shape. The substantially circular array has a diameter of about 7 mm, and is arranged substantially centrally in the element 301.

The passages (not shown) have a substantially circular cross-section and are tapered from the inlet side 308 to the outlet side 309 of the vibratable element 301. The passages have a diameter at the inlet side of about 8 µm and a diameter at the outlet side of about 6 µm. The passages are typically formed by high-speed laser drilling. The plurality of passages is comprised of about 4000 passages arranged with equal spacing across the array.

Actuator 302 comprises a piezoelectric transducer. The piezoelectric transducer is a substantially circular annular disc of piezoelectric material, typically zirconate titanate (PZT). The piezoelectric transducer has a thickness of about 2 mm, an outer diameter of about 17 mm and an inner diameter of about 8 mm.

As shown in Figure 2, the actuator 302 is in direct contact with the vibratable element 301, at the outlet side 309 of the vibratable element. The inner diameter of the piezoelectric transducer 302 circumscribes the array of passages 303 of the vibratable element 301, such that the open ends of the passages at the outlet side are not covered by the piezoelectric transducer 302. In other embodiments (not shown) it is envisaged that the piezoelectric transducer 302 may be in direct contact with the vibratable element 301 at the inlet side 308.

The vibratable element 301 and piezoelectric transducer 302 are supported within the atomiser housing 304 by a pair of elastomeric O-rings 311, which allow the vibratable element 301 and the piezoelectric transducer 302 to vibrate within the housing 304. The vibratable element 301 and piezoelectric transducer 302 are held together by pressure from the opposing O-rings 311. However, in other embodiments (not shown) the vibratable element 301 and the piezoelectric transducer 302 may be bonded by any suitable means, such as an adhesive layer.

The vibratable element 301 and the piezoelectric transducer 302 are arranged within the atomiser housing 304 such that the array of passages 303 is in coaxial alignment with the inlet and outlet openings 305, 306 of the housing 304.

One or more spring pins 310 extend through an opening 312 in the atomiser housing 304 to provide electrical connection of the piezoelectric transducer 302 to the control electronics 104 and the battery 102 of the device 100. The one or more spring pins 310 are held in contact with the piezoelectric transducer 302 by pressure, rather than by a mechanical connection so that good electrical contact is maintained during vibration of the piezoelectric transducer 302.

In use, when the atomiser 300 is removably connected to the mouthpiece portion 120 of the device 100 and the cartridge 200 is received in the cavity 112 of the device, an elongate capillary body (not shown in Figure 1) extends from the liquid storage portion 201 of the cartridge 200 to the atomiser 300 to fluidly connect the cartridge 200 to the atomiser 300. As shown in Figure 2, the elongate capillary body 204 extends into the atomiser housing 304 and abuts the inlet side 308 of the vibratable element 301 at the array of passages 303. Heating means is provided in the liquid storage portion in the form of a coil heater 205 surrounding the capillary body 204. Note that the coil heater is only shown schematically in Figure 2. The coil heater 205 is connected to the electric circuitry 104 and battery 102 of the device 100 via connections (not shown), which may pass along the outside of the liquid storage portion 200, although this is not shown in Figure 1 or Figure 2.

In use, liquid aerosol-forming substrate (not shown) is conveyed by capillary action from the liquid storage portion 201 from the end of the capillary body 204 which extends into the liquid storage portion 201, past the heater coil 205, and to the other end of the capillary body 204, which extends into the atomiser housing 304 and abuts the vibratable element 301 at the inlet side 308 at the array of passages 303.

When a user draws on the air outlet 124 of the mouthpiece portion 120, ambient air is drawn through air inlets 122. In the embodiment of Figure 1, a puff detection device 106 in the form of a microphone, is also provided as part of the control electronics 104. A small air flow is drawn through a sensor inlet 121 in the main body housing 101, past the microphone 106 and up into the mouthpiece portion 120. When a puff is detected by the electric circuitry 104, the electric circuitry 104 activates the heater coil 205 and the piezoelectric transducer 302. The battery 102 supplies electrical energy to the coil heater 205 to heat the capillary body 204 surrounded by the coil heater. The battery 102 further supplies electrical energy to the piezoelectric transducer 302, which vibrates, deforming in the thickness direction. The piezoelectric transducer 302 typically vibrates at approximately 150 kHz. The piezoelectric transducer 302 transmits the vibrations to the vibratable element 301, which vibrates, also deforming in the thickness direction. An LED 108 is also activated to indicate that the device is activated.

The coil heater 205 heats the liquid aerosol-forming substrate being conveyed along the capillary body, past the coil heater 205, to a predetermined temperature of about 45°C.

The vibrations in the vibratable element deform the plurality of passages 303, which draws heated liquid aerosol-forming substrate from the capillary body 204, through the plurality of passages 303 at the inlet side 308 of the vibratable element 301, and ejects atomised droplets of liquid aerosol-forming substrate from the passages at the outlet side 309 of the vibratable element 301, forming an aerosol. At the same time, the heated liquid being atomised is replaced by further liquid moving along the capillary body 204 by capillary action. (This is sometimes referred to as pumping action'). The aerosol droplets ejected from the vibratable element 301 mix with and are carried in the air flow 127 from the inlets 122 in the aerosol forming chamber 125, and are carried towards the air outlet 124 of the mouthpiece 120 for inhalation by the user.

In the embodiment shown in Figure 1, the electric circuitry 104 is programmable, and can be used to manage the aerosol generating operation.

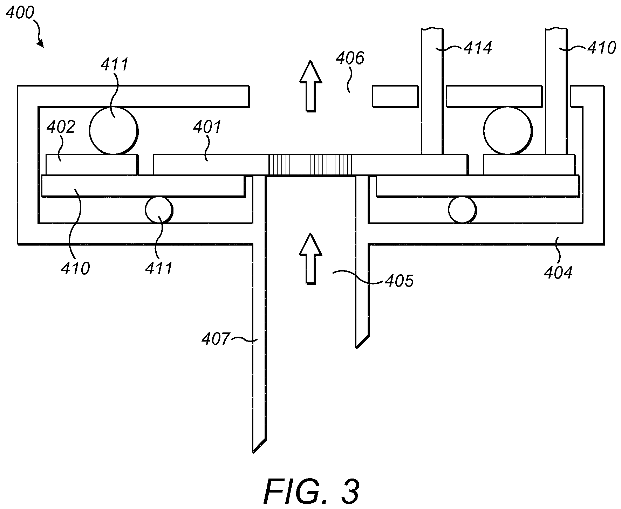

Another embodiment of an atomiser for use in the system of Figure 1, is shown in Figure 3. The atomiser 400 has a similar construction and size to the atomiser 300 shown in Figure 2. However, the atomiser 400 is provided with a washer 410 within the housing 404, on which the vibratable element 401 and the piezoelectric transducer 402 are supported. The washer 410 transmits vibrations from the piezoelectric element 402 to the vibratable element 401.

In this embodiment, the vibratable element has a smaller diameter of about 12 mm. The actuator is not arranged over the vibratable element and, therefore, has a larger inner diameter of about 14 mm.

The washer 410 is a substantially circular annular disc, having a thickness of about 2 mm, an outer diameter of about 17 mm and an inner diameter of about 10 mm. The vibratable element 401 and the piezoelectric transducer are bonded to one side of the washer 410 by an adhesive layer (not shown). The washer 410 is bonded to the vibratable element 401 at the inlet side 408. The piezoelectric transducer 402 substantially circumscribes the vibratable element 401. A pair of O-rings 411, similar to the pair of O-rings 311 of the atomiser 300 shown in Figure 2, supports the vibratable element 401, the piezoelectric transducer 402 and the washer 410 in the atomiser housing 404.

In other embodiments (not shown) the vibratable element 401, the piezoelectric transducer 402 and the washer 410 may be arranged differently. The washer 410 may be bonded to the vibratable element 401 at the outlet side 409. The vibratable element 401 may be secured to the washer 410 at the opposite side to the piezoelectric transducer 402.

The piezoelectric transducer is electrically connected to the control electronics 104 and the battery 102 of the device 100 by one or more spring pins 410 extending through one or more openings in the atomiser housing 404.

In this embodiment, the vibratable element 401 is also electrically connected to the control electronics 104 and the battery 102 of the device 100, so that the vibratable element 401 may form a resistive heating element. As such, the heating means of the system comprises the vibratable element 401. Electrical connection of the vibratable element 401 with the control electronics 104 and the battery 102 is achieved by one or more second spring pins 414 extending through one or more openings in the housing 404 of the atomiser 400. The one or more second spring pins 414 are held in contact with the vibratable element 401 by pressure, rather than by a mechanical connection so that a the electrical connection remains during vibration of the vibratable element 401.

In this embodiment, a capillary body does not fluidly connect the atomiser 400 to the liquid storage portion 201 of the cartridge 200. Instead, liquid aerosol-forming substrate (not shown) is free to flow from the liquid storage portion 201 of the cartridge 200 to the vibratable element 401 of the atomiser 400 via an inlet opening 405 in the atomiser housing 404. The housing 404 of the atomiser 400 further comprises piercing means 401, for piercing a lid of a cartridge (not shown) on insertion of a sealed cartridge into the device 100. The piercing element 407 is a substantially circularly cylindrical tube arranged to guide liquid aerosol-forming substrate from the liquid storage portion (not shown) to the vibratable element 401. The piercing element 407 extends into the housing 404 from the inlet opening 405 to the inlet side of the vibratable element 401. The piercing element 407 extends out of the housing from the inlet opening 405 by about 6 mm. The distal end of the piercing element is angled to form a sharp point to facilitate piercing of a cartridge lid.

In use, the atomiser 400 operates in a substantially similar manner to the atomiser 300 shown in Figure 2. However, power is supplied from the battery 102 to the vibratable element 401 for heating the vibratable element 401, and free flowing liquid aerosol-forming substrate (not shown) at the inlet side of the vibratable element 401 is heated by the vibratable element 401 to a predetermined temperature of about 45°C. Heated liquid aerosol-forming substrate at the inlet side of the vibratable element 401 is drawn into the plurality of passages by the vibrations of the vibratable element 401, passes through the passages and is atomised substantially as described above and exits the atomiser 400 via the outlet opening 406 in the atomiser housing 404.

In other embodiments (not shown), a plunger or other similar type of device may be provided at the end of the cartridge 200 opposite the opening, such that liquid aerosol-forming substrate may be moved into contact with the vibratable element, regardless of the orientation of the device.

In other embodiments (not shown) the heating means may not comprise the vibratable element, but rather heating means may be provided at another location in or on the atomiser 400. For example, heating means may be provided in or on the atomiser housing 404, on or around the piercing element 407, on the washer 410 of the atomiser 400 or on the vibratable element 401. Where the heating means is provided on the vibratable element 401, the vibratable element may be heated by the heating means to further facilitate heating of the liquid aerosol-forming substrate. The heating means may be any suitable heating means, as described in more detail above.

In other embodiments (not shown) the atomiser 300 may be removably connected to the main body housing 101 of the device 100. The atomiser 300 may be arranged in the cavity 112 for receiving the cartridge 200. The atomiser may be arranged at the distal end of the cavity 112, such that the cartridge 200 may be inserted and removed from the main body at the mouthpiece end. One or more air inlets 122 may be arranged distally of the vibratable element in the main body housing 101, and an airflow pathway may be provided between the air inlets 122, the atomiser 300 and the output 124 of the mouthpiece 120, such that when a user draws on the mouthpiece 120, air enters the main body housing 101 at the one or more air inlets 122, passes over the atomiser 300, entraining aerosol generated by the atomiser, and passes through the device 100 to the mouthpiece for inhalation by the user.

In other embodiments (not shown) the cartridge may comprise the atomiser 300, including the vibratable element 301 and the piezoelectric transducer 302. Contacts may be provided in the cartridge 200 and in the device 100 to connect the control electronics 104 and the battery 102 to the atomiser 300 in the cartridge 200.

In other embodiments (not shown) the device 100 may include one or more secondary air inlets arranged to draw in additional ambient air to reduce the temperature of the aerosol entrained in the airflow and to dilute the aerosol before inhalation by the user.

In other embodiments (not shown) the heating means may be inductive heating means, such that no electrical contacts are formed between the cartridge and the device. The cartridge may comprise a susceptor element positioned to heat the aerosol-forming substrate. The device may comprise an inductor coil and the control electronics 104 and power supply 102 may be configured to provide high frequency oscillating current to the inductor coil to induce current in the susceptor element.

In other embodiments (not shown) the heating means may be provided in the cavity 112 of the device 100.

## Claims

1. An aerosol-generating system comprising:
a liquid-storage portion (201) comprising a housing for holding a liquid aerosol-forming substrate;
heating means arranged to heat liquid aerosol-forming substrate, the heating means comprising means for heating a small amount of liquid aerosol-forming substrate at a time, the means for heating a small amount of liquid aerosol-forming substrate at a time comprising:
a liquid passageway in communication with the liquid aerosol-forming substrate; and
at least one heater arranged to heat liquid aerosol-forming substrate
within the liquid passageway;
a vibratable element (301) comprising a plurality of passages (303) through which heated liquid aerosol-forming substrate passes to form an aerosol; and
an actuator (302) arranged to vibrate the vibratable element (301) to generate the aerosol.

2. An aerosol-generating system as claimed in claim 1, wherein the heating means is arranged on or within the housing of the liquid-storage portion (201).

3. An aerosol-generating system as claimed in claim 1, wherein the heating means is arranged at the vibratable element (301).

4. An aerosol-generating system as claimed in claim 1, wherein the heating means comprises the vibratable element (301).

5. An aerosol-generating system as claimed in any preceding claim, wherein the aerosol-generating system further comprises a control system configured to operate the heating means to heat aerosol-forming substrate to a predetermined temperature.

6. An aerosol-generating system as claimed in any preceding claim, wherein the vibratable element (301) comprises an inlet side (308) and an opposing outlet side (309), and wherein each passage of the plurality of passages (303) extends from the inlet side (308) to the outlet side (309).

7. An aerosol-generating system as claimed in claim 6, wherein the actuator (302) is arranged to transmit vibrations to the vibratable element (301) to the inlet side (308) or the outlet side (309) of the vibratable element (301).

8. An aerosol-generating system as claimed in any preceding claim, wherein the actuator (302) comprises a piezoelectric transducer.

9. An aerosol-generating system as claimed in any preceding claim, wherein the liquid-storage portion (201) further comprises a carrier material within the housing for holding the aerosol-forming substrate.

10. An aerosol-generating system as claimed in any preceding claim, wherein the at least one heater comprises a coil (205) substantially surrounding at least a portion of a liquid passageway.

11. An aerosol-generating system as claimed in any preceding claim, further comprising a liquid aerosol-forming substrate in the housing of the liquid-storage portion (201).

12. An aerosol-generating system as claimed in any preceding claim, wherein the system further comprises a cartridge (200), wherein the cartridge (200) comprises the liquid-storage portion (201).

13. An aerosol-generating system as claimed in any preceding claim, wherein the system is an electrically operated smoking system.

14. A cartridge (200) for an aerosol-generating system, the cartridge (200) comprising:
a liquid-storage portion (201) comprising a housing for holding a liquid aerosol-forming substrate;
heating means arranged to heat liquid aerosol-forming substrate to a predetermined temperature, the heating means comprising means for heating a small amount of liquid aerosol-forming substrate at a time, the means for heating a small amount of liquid aerosol-forming substrate at a time comprising:
a liquid passageway in communication with the liquid aerosol-forming substrate; and
at least one heater arranged to heat liquid aerosol-forming substrate
within the liquid passageway; and
a vibratable element (301) comprising a plurality of passages (303) through which heated liquid aerosol-forming substrate passes to form an aerosol.

15. An atomiser (300) for atomising a liquid aerosol-generating substrate to generate an aerosol, the atomiser (300) comprising:
heating means arranged to heat liquid aerosol-forming substrate, the heating means comprising means for heating a small amount of liquid aerosol-forming substrate at a time, the means for heating a small amount of liquid aerosol-forming substrate at a time comprising:
a liquid passageway for communication with a liquid aerosol-forming substrate; and
at least one heater arranged to heat liquid aerosol-forming substrate
within the liquid passageway;
a vibratable element (301) comprising a plurality of passages (303) through which heated liquid aerosol-forming substrate passes to form an aerosol; and
an actuator (302) arranged to vibrate the vibratable element (301) to generate the aerosol.

16. A method of generating an aerosol, the method comprising:
providing a liquid passageway for communication of liquid aerosol-forming substrate;
providing at least one heater arranged to head liquid aerosol-forming substrate within the liquid passageway;
heating liquid aerosol-forming substrate in the liquid passageway to a predetermined temperature;
receiving liquid aerosol-forming substrate heated to the predetermined temperature at a vibratable element (301) having a plurality of passages (303); and
vibrating the vibratable element (301) to move liquid aerosol-forming substrate through the passages (303) to form an aerosol.

## Patentansprüche

1. Aerosolerzeugungssystem, aufweisend:
einen Flüssigspeicherteil (201), der ein Gehäuse zur Aufnahme eines flüssigen aerosolbildenden Substrats aufweist;
Heizmittel, angeordnet zum Erwärmen eines flüssigen aerosolbildenden Substrats, wobei das Heizmittel Mittel zum Erwärmen einer jeweils kleinen Menge eines flüssigen aerosolbildenden Substrats aufweist, wobei das Mittel zum Erwärmen einer jeweils kleinen Menge eines flüssigen aerosolbildenden Substrats Folgendes aufweist:
einen Flüssigkeitsdurchgang in Verbindung mit dem flüssigen aerosolbildenden Substrat; und
zumindest eine Heizvorrichtung, die angeordnet ist, um ein flüssiges aerosolbildendes Substrat innerhalb des Flüssigkeitsdurchgangs zu erwärmen;
ein vibrierfähiges Element (301), das eine Vielzahl von Durchgängen (303) aufweist, durch die das erwärmte flüssige aerosolbildende Substrat strömt, um ein Aerosol zu bilden; und
ein Stellglied (302), das so angeordnet ist, dass es das vibrierfähige Element (301) vibriert, um das Aerosol zu erzeugen.

2. Aerosolerzeugungssystem nach Anspruch 1, wobei das Heizmittel auf oder innerhalb des Gehäuses des Flüssigspeicherteils (201) angeordnet ist.

3. Aerosolerzeugungssystem nach Anspruch 1, wobei das Heizmittel an dem vibrierfähigen Element (301) angeordnet ist.

4. Aerosolerzeugungssystem nach Anspruch 1, wobei das Heizmittel das vibrierfähige Element (301) aufweist.

5. Aerosolerzeugungssystem nach einem der vorhergehenden Ansprüche, wobei das Aerosolerzeugungssystem ferner ein Steuersystem aufweist, das so ausgelegt ist, dass es das Heizmittel zum Erwärmen des aerosolbildenden Substrats auf eine vorbestimmte Temperatur betreibt.

6. Aerosolerzeugungssystem nach einem der vorhergehenden Ansprüche, wobei das vibrierfähige Element (301) eine Einlassseite (308) und eine gegenüberliegende Auslassseite (309) aufweist und wobei jeder Durchgang der Vielzahl von Durchgängen (303) sich von der Einlassseite (308) zur Auslassseite (309) erstreckt.

7. Aerosolerzeugungssystem nach Anspruch 6, wobei das Stellglied (302) so angeordnet ist, dass es Vibrationen auf dem vibrierfähigen Element (301) auf die Einlassseite (308) oder die Auslassseite (309) des vibrierfähigen Elements (301) überträgt.

8. Aerosolerzeugungssystem nach einem der vorhergehenden Ansprüche, wobei das Stellglied (302) einen piezoelektrischen Wandler aufweist.

9. Aerosolerzeugungssystem nach einem der vorhergehenden Ansprüche, wobei der Flüssigspeicherteil (201) ferner ein Trägermaterial innerhalb des Gehäuses zur Aufnahme des aerosolbildenden Substrats aufweist.

10. Aerosolerzeugungssystem nach einem der vorhergehenden Ansprüche, wobei die zumindest eine Heizvorrichtung eine Spule (205) aufweist, die im Wesentlichen zumindest einen Teil eines Flüssigkeitsdurchgangs umgibt.

11. Aerosolerzeugungssystem nach einem der vorhergehenden Ansprüche, ferner aufweisend ein flüssiges aerosolbildendes Substrat im Gehäuse des Flüssigspeicherteils (201).

12. Aerosolerzeugungssystem nach einem der vorhergehenden Ansprüche, wobei das System ferner eine Patrone (200) aufweist, wobei die Patrone (200) den Flüssigspeicherteil (201) aufweist.

13. Aerosolerzeugungssystem nach einem der vorhergehenden Ansprüche, wobei das System ein elektrisch betriebenes Rauchsystem ist.

14. Patrone (200) für ein Aerosolerzeugungssystem, wobei die Patrone (200) aufweist:
einen Flüssigspeicherteil (201), der ein Gehäuse zur Aufnahme eines flüssigen aerosolbildenden Substrats aufweist;
Heizmittel, angeordnet zum Erwärmen eines flüssigen aerosolbildenden Substrats auf eine vorbestimmte Temperatur, wobei das Heizmittel Mittel zum Erwärmen einer jeweils kleinen Menge eines flüssigen aerosolbildenden Substrats aufweist, wobei das Mittel zum Erwärmen einer jeweils kleinen Menge eines flüssigen aerosolbildenden Substrats Folgendes aufweist:
einen Flüssigkeitsdurchgang in Verbindung mit dem flüssigen aerosolbildenden Substrat; und
zumindest eine Heizvorrichtung, die angeordnet ist, um ein flüssiges aerosolbildendes Substrat innerhalb des Flüssigkeitsdurchgangs zu erwärmen; und
ein vibrierfähiges Element (301), das eine Vielzahl von Durchgängen (303) aufweist, durch die das erwärmte flüssige aerosolbildende Substrat strömt, um ein Aerosol zu bilden.

15. Ein Zerstäuber (300) zum Zerstäuben eines flüssigen aerosolerzeugenden Substrats zur Erzeugung eines Aerosols, wobei der Zerstäuber (300) aufweist:
Heizmittel, angeordnet zum Erwärmen eines flüssigen aerosolbildenden Substrats, wobei das Heizmittel Mittel zum Erwärmen einer jeweils kleinen Menge eines flüssigen aerosolbildenden Substrats aufweist, wobei das Mittel zum Erwärmen einer jeweils kleinen Menge eines flüssigen aerosolbildenden Substrats Folgendes aufweist:
einen Flüssigkeitsdurchgang zur Verbindung mit einem flüssigen aerosolbildenden Substrat; und
zumindest eine Heizvorrichtung, die angeordnet ist, um ein flüssiges aerosolbildendes Substrat innerhalb des Flüssigkeitsdurchgangs zu erwärmen;
ein vibrierfähiges Element (301), das eine Vielzahl von Durchgängen (303) aufweist, durch die das erwärmte flüssige aerosolbildende Substrat strömt, um ein Aerosol zu bilden; und
ein Stellglied (302), das so angeordnet ist, dass es das vibrierfähige Element (301) vibriert, um das Aerosol zu erzeugen.

16. Verfahren zum Erzeugen eines Aerosols, wobei das Verfahren Folgendes aufweist:
Bereitstellen eines Flüssigkeitsdurchgangs zur Verbindung mit einem flüssigen aerosolbildenden Substrat;
Bereitstellen zumindest einer Heizvorrichtung, die angeordnet ist, um ein flüssiges aerosolbildendes Substrat innerhalb des Flüssigkeitsdurchgangs zu erwärmen;
Erwärmen des flüssigen aerosolbildenden Substrats in dem Flüssigkeitsdurchgang auf eine vorbestimmte Temperatur; Empfangen des flüssigen aerosolbildenden Substrats, das auf die vorbestimmte Temperatur erwärmt wurde, an einem vibrierfähigen Element (301), das eine Vielzahl von Durchgängen (303) hat; und
Vibrieren des vibrierfähigen Elements (301), um ein flüssiges aerosolbildendes Substrat durch die Durchgänge (303) zu bewegen, um ein Aerosol zu bilden.

## Revendications

1. Système générateur d'aérosol comprenant :
une partie de stockage de liquide (201) comprenant un logement pour contenir un substrat liquide formant aérosol ;
des moyens de chauffage agencés pour chauffer le substrat liquide formant aérosol, les moyens de chauffage comprenant des moyens pour chauffer une petite quantité de substrat liquide formant aérosol à la fois, les moyens pour chauffer une petite quantité de substrat liquide formant aérosol à la fois, comprenant :
un passage de liquide en communication avec le substrat liquide formant aérosol ; et
au moins un dispositif de chauffage agencé pour chauffer le substrat liquide formant aérosol à l'intérieur du passage de liquide ;
un élément vibrant (301) comprenant une pluralité de passages (303) à travers lesquels le substrat liquide formant aérosol chauffé passe pour former un aérosol ; et
un actionneur (302) agencé pour faire vibrer l'élément vibrant (301) pour générer l'aérosol.

2. Système générateur d'aérosol selon la revendication 1, dans lequel les moyens de chauffage sont agencés sur ou à l'intérieur du logement de la partie de stockage de liquide (201) .

3. Système générateur d'aérosol selon la revendication 1, dans lequel les moyens de chauffage sont agencés au niveau de l'élément vibrant (301).

4. Système générateur d'aérosol selon la revendication 1, dans lequel les moyens de chauffage comprennent l'élément vibrant (301) .

5. Système générateur d'aérosol selon l'une quelconque des revendications précédentes, dans lequel le système générateur d'aérosol comprend en outre un système de contrôle configuré pour faire fonctionner les moyens de chauffage pour chauffer le substrat formant aérosol à une température prédéterminée.

6. Système générateur d'aérosol selon l'une quelconque des revendications précédentes, dans lequel l'élément vibrant (301) comprend un côté d'entrée (308) et un côté de sortie opposé (309), et dans lequel chaque passage de la pluralité de passages (303) s'étend depuis le côté d'entrée (308) au côté de sortie (309).

7. Système générateur d'aérosol selon la revendication 6, dans lequel l'actionneur (302) est agencé pour transmettre des vibrations à l'élément vibrant (301) vers le côté d'entrée (308) ou le côté de sortie (309) de l'élément vibrant (301).

8. Système générateur d'aérosol selon l'une quelconque des revendications précédentes, dans lequel l'actionneur (302) comprend un transducteur piézoélectrique.

9. Système générateur d'aérosol selon l'une quelconque des revendications précédentes, dans lequel la partie de stockage de liquide (201) comprend en outre un matériau porteur à l'intérieur du logement pour contenir le substrat formant aérosol.

10. Système générateur d'aérosol selon l'une quelconque des revendications précédentes, dans lequel l'au moins un dispositif de chauffage comprend une bobine (205) entourant sensiblement au moins une partie d'un passage de liquide.

11. Système générateur d'aérosol selon l'une quelconque des revendications précédentes, comprenant en outre un substrat liquide formant aérosol dans le logement de la partie de stockage de liquide (201).

12. Système générateur d'aérosol selon l'une quelconque des revendications précédentes, dans lequel le système comprend en outre une cartouche (200), dans lequel la cartouche (200) comprend la partie de stockage de liquide (201).

13. Système générateur d'aérosol selon l'une quelconque des revendications précédentes, dans lequel le système est un système de fumage à commande électrique.

14. Cartouche (200) pour un système générateur d'aérosol, la cartouche (200) comprenant :
une partie de stockage de liquide (201) comprenant un logement pour contenir un substrat liquide formant aérosol ;
des moyens de chauffage agencés pour chauffer le substrat liquide formant aérosol à une température prédéterminée, les moyens de chauffage comprenant des moyens pour chauffer une petite quantité de substrat liquide formant aérosol à la fois, les moyens pour chauffer une petite quantité de substrat liquide formant aérosol à la fois, comprenant :
un passage de liquide en communication avec le substrat liquide formant aérosol ; et
au moins un dispositif de chauffage agencé pour chauffer le substrat liquide formant aérosol dans le passage de liquide ; et
un élément vibrant (301) comprenant une pluralité de passages (303) à travers lesquels le substrat liquide formant aérosol chauffé passe pour former un aérosol.

15. Atomiseur (300) pour l'atomisation d'un substrat liquide générateur d'aérosol pour générer un aérosol, l'atomiseur (300) comprenant :
des moyens de chauffage agencés pour chauffer le substrat liquide formant aérosol, les moyens de chauffage comprenant des moyens pour chauffer une petite quantité de substrat liquide formant aérosol à la fois, les moyens pour chauffer une petite quantité de substrat liquide formant aérosol à la fois, comprenant :
un passage de liquide pour la communication avec un substrat liquide formant aérosol ; et
au moins un dispositif de chauffage agencé pour chauffer le substrat liquide formant aérosol à l'intérieur du passage de liquide ;
un élément vibrant (301) comprenant une pluralité de passages (303) à travers lesquels le substrat liquide formant aérosol chauffé passe pour former un aérosol ; et
un actionneur (302) agencé pour faire vibrer l'élément vibrant (301) pour générer l'aérosol.

16. Procédé de génération d'un aérosol, le procédé comprenant :
la fourniture d'un passage de liquide pour la communication du substrat liquide formant aérosol ;
la fourniture d'au moins un dispositif de chauffage agencé pour diriger le substrat liquide formant aérosol à l'intérieur du passage de liquide ;
le chauffage du substrat liquide formant aérosol dans le passage de liquide à une température prédéterminée ;
la réception d'un substrat liquide formant aérosol chauffé à la température prédéterminée au niveau d'un élément vibrant (301) ayant une pluralité de passages (303) ; et
la vibration de l'élément vibrant (301) pour déplacer le substrat liquide formant aérosol à travers les passages (303) pour former un aérosol.
